# EUROPEAN PATENT SPECIFICATION

(11) **EP 2 413 888 B1**
(45) Date of publication and mention of the grant of the patent: **05.09.2018**
(21) Application number: 09789567.6
(22) Date of filing: 02.04.2009
(51) Int. Cl.: A61K 8/34, A61K 8/27, A61K 8/73, A61K 8/81, A61Q 11/00

(54) **DENTIFRICE COMPOSITION**
ZAHNPASTAZUSAMMENSETZUNG
COMPOSITION DE DENTIFRICE

(43) Date of publication of application: 08.02.2012
(73) Proprietor: Colgate-Palmolive Company, New York, NY 10022 (US)
(72) Inventor: NOWAK, Andrew, Piscataway NJ 08854 (US); CAMPBELL, Thomas, S., Flemington New Jersey 08822 (US); FISHER, Steven, Middlesex NJ 08846 (US)
(74) Representative: Wibbelmann, Jobst
(86) International application number: PCT/US2009/039268
(87) International publication number: WO 2010/114546

(56) References cited:
- EP-A- 1 837 009
- WO-A-92/10994
- WO-A-2007/076444
- US-A- 5 500 448
- US-A1- 2003 124 067

## Description

### BACKGROUND OF THE INVENTION

Metal ions such as zinc ions are known to be effective anti-microbial agents. These metal ions provide anti-gingivitis and anti-plaque benefits and may also improve breath and reduce sensitivity. In particular, zinc citrate has been shown to have anti-plaque, anti-gingivitis and anti-tartar efficacy. In addition, zinc has also shown its efficacy as an anti-malodor agent.

There is a need to develop dentifrices that provide multiple therapeutic benefits by combining zinc ions with other actives in a single composition.

However, dentifrice compositions containing, in combination, zinc cations, maleic anhydride copolymer as an antibacterial enhancing agent for triclosan, and thickening agents such as carboxymethyl cellulose can suffer from the technical problem of poor rheological properties. In particular, the dentifrice composition can exhibit an unacceptably high viscosity, which can make the composition difficult to dispense from the dispenser and difficult to disperse in the mouth during tooth brushing, resulting in a poor feeling in the mouth for the consumer. Such dentifrice compositions can also tend to exhibit progressive thickening over time. If a formulation routinely increases in viscosity over time, dispensing of the formulation will become difficult, which will likely result in consumer dissatisfaction. US 5,500,448 discloses the use of triclosan in the manufacture of a composition for the prevention, inhibition and/or reduction of aphthous ulcers. WO 92/10994 discloses oral compositions which contain a noncationic water insoluble anti-bacterial agent and an alkali metal or ammonium zinc salt, which are effective against plaque and gingivitis. WO2007/076444 describes improved oral compositions comprising zinc acetate and/or tocopherol agents.

### SUMMARY OF THE INVENTION

The present invention relates to dentifrice compositions comprising zinc ions.

One aim of the present invention is to provide a dentifrice composition containing, in combination, zinc cations, maleic anhydride copolymer as an antibacterial enhancing agent for triclosan, and at least one thickening agent which can exhibit good rheological properties.

Another aim of the present invention is to provide such a dentifrice composition which has a viscosity which permits the composition to be readily dispensable from a dispenser, such as a tube or pump, and readily dispersable in the mouth during tooth brushing.

A further aim of the present invention is to provide such a dentifrice composition which can comprise a range of amounts of zinc cations within the composition without a significant variation in the viscosity of the composition, which viscosity permits the composition to be readily dispensable from a dispenser.

A yet further aim of the present invention is to provide such a dentifrice composition containing zinc cations which tends not to exhibit progressive thickening over time.

The present invention specifically provides a dentifrice composition comprising: a. an orally acceptable vehicle; b. a zinc salt of an organic acid as a source of zinc ions, wherein the zinc salt is present in an amount of from 1 to 2.5 wt % based on the weight of the composition; c. a polysaccharide thickening agent, wherein the polysaccharide thickening agent consists of xanthan gum, and the xanthan gum is present in an amount of from 0.5 to 1.5 wt % based on the weight of the composition; d. triclosan as an antibacterial agent, and e. a methyl vinyl ether/maleic anhydride copolymer as an enhancing agent for the anti-bacterial agent.

### DETAILED DESCRIPTION

There is a need in the art to provide oral care compositions for the delivery to and uptake by dental tissue of antibacterial compounds contained in oral compositions containing zinc ions, to provide therapeutic efficacy of the antibacterial agent and of the zinc ions.

There is a desire to utilize zinc salts in combination with triclosan to provide improved efficacy over current triclosan-containing dentifrice formulations in the areas of tartar control, fresh breath benefits and plaque/gingivitis reduction.

As will be demonstrated herein, the preferred embodiments of the present invention can provide a dentifrice that provides multiple therapeutic benefits by combining zinc ions, e.g. as zinc citrate, and triclosan in combination with an antibacterial enhancing agent for triclosan. The dentifrice contains thickening agents, yet exhibits acceptable rheological properties.

The preferred embodiments of the present invention can also provide that the initial viscosity of the dentifrice compositions is acceptable and permits ready dispensing of the dentifrice composition from a dispenser, and also that the dentifrice composition has a stable rheology which does not tend progressively to thicken over time but instead quickly, for example within a few days of manufacture, reaches a stable viscosity.

All percentages used herein are by weight of the dentifrice composition, unless otherwise specified. All measurements are made at 25[deg.] C., unless otherwise specified.

Herein, "comprising" means that other steps and other components which do not affect the end result can be added. This term encompasses the terms "consisting of" and "consisting essentially of." Herein, "effective amount" means an amount of a compound or composition sufficient to significantly induce a positive benefit, preferably an oral health benefit, but low enough to avoid serious side effects, i.e., to provide a reasonable benefit to risk ratio, within the sound judgment of a skilled artisan.

The dentifrice composition of the present invention may be in the form of a toothpaste or dentifrice. The term "dentifrice", as used herein, means paste or gel formulations unless otherwise specified. The dentifrice composition may be in any desired form, such as deep striped, surface striped, multi-layered, having the gel surrounding the paste, or any combination thereof.

The dentifrice composition is a product, which in the ordinary course of administration, is not intentionally swallowed for purposes of systemic administration of particular therapeutic agents, but is rather retained in the oral cavity for a time sufficient to contact substantially all of the tooth surfaces and/or oral tissues for purposes of oral activity.

The term "aqueous carrier" as used herein means any safe and effective materials for use in the compositions of the present invention. Such materials include thickening agents, humectants, ionic active ingredients, buffering agents, anticalculus agents, abrasive polishing materials, peroxide sources, alkali metal bicarbonate salts, surfactants, titanium dioxide, coloring agents, flavor systems, sweetening agents, antimicrobial agents, herbal agents, desensitizing agents, stain reducing agents, and mixtures thereof.

In a first aspect, the invention provides a dentifrice composition comprising: a. an orally acceptable vehicle; b. a zinc salt of an organic acid as a source of zinc ions, wherein the zinc salt is present in an amount of from 1 to 2.5 wt % based on the weight of the composition; c. a polysaccharide thickening agent, wherein the polysaccharide thickening agent consists of xanthan gum, and the xanthan gum is present in an amount of from 0.5 to 1.5 wt % based on the weight of the composition; d. triclosan as an antibacterial agent, and e. a methyl vinyl ether/maleic anhydride copolymer as an enhancing agent for the anti-bacterial agent.

The present invention may include dentifrice formulations containing adherent agent, as set forth in detail elsewhere herein. With a maleic anhydride copolymer, such as the Gantrez polymer, zinc cations tend to react with certain thickening agents that may be present in the dentifrice formulation, in particular such thickening agents which are present as hydrocolloids and have a high concentration of groups, such as carboxylate groups, which are available to react by cross-linking with zinc cations. Such reactions tend to form an ionic bridge between the maleic anhydride copolymer and, for example, any cellulosic polymer present in the dentifrice composition as thickening agent. An unacceptably high viscosity in the bulk formula results from this interaction.

The present inventors have found, however, that the use of a thickening agent that has a low concentration of reactive groups, for example carboxylate groups, such as xanthan gum, either as the sole thickening agent or as one of a plurality of thickening agents, for the maleic anhydride copolymer system provides fewer sites for chemical interaction between the zinc ions and the maleic anhydride copolymer. This in turn allows for the incorporation of higher levels of the zinc salt, e.g. zinc citrate, than would be possible using other thickening systems, for example based on carboxymethyl cellulose (CMC) or CMC/Carrageenan formulas, without unacceptable increases in viscosity.

In accordance with the preferred embodiments of the present invention, a dentifrice containing both zinc cations and triclosan can be formulated provided to provide two actives having different and complementary methods of action, and this can be achieved by use of a dentifrice gum system which includes, either wholly or partly, xanthan gum. The dentifrice can employ high levels of the zinc active, for example by using zinc citrate at a relatively high level of from 1 to 2% by weight, based on the weight of the composition, without detrimental effects to the product aesthetics as a result of an unacceptable increase in viscosity. A particularly preferred amount of zinc citrate for use against plaque and gingivitis is 2% by weight, based on the weight of the composition.

Without being bound by any theory, the present inventors believe that any compatibility between zinc cations and a maleic anhydride copolymer system is significantly reduced when certain hydrocolloids are also present in the composition. This is due to ionic bridging occurring between the maleic anhydride copolymer and the hydrocolloid, which is facilitated by the divalent cationic nature of the zinc cations. Carboxymethyl cellulose (CMC), which is such a hydrocolloid employed as a thickener, is especially prone to this binding due to the amount and nature of the substituted groups along the backbone. In order to use zinc in the presence of triclosan and maleic anhydride copolymer, such as the Gantrez product, this reaction must be removed or limited.

Both CMC and xanthan gum contain carboxylate groups along their backbones. While both materials are charged polysaccharides, the density of charged carboxylate groups along the backbone is quite different, and much higher for the CMC than for the xanthan gum. For example, one known commercially available CMC, CMC 2000S (available from CPKelco) has a degree of substitution of ∼0.9 carboxylate groups per sugar residue. Xanthan gum, in comparison, has a degree of substitution of <0.4 carboxylate groups per sugar residue. The number of carboxylate groups associated with the chain is important to the rheological properties of a mixture that contains divalent ions such as Zn²⁺ due to the fact that these ions complex with the carboxylate groups to form ionic crosslinks or bridges, with the zinc ions crosslinked to two opposing carboxylate groups.

These linkages can be formed from not only carboxylate groups found on CMC, but also sulfate groups found in other thickening agents such as i-Carrageenan. Such linkages can lead to interchain association of chains and the formation of large networks that can act to dramatically increase viscosity or solubility of the polymer species.

In part, the present inventors have found that a substitution of xanthan for CMC in a dentifrice can reduce the number of ionic linkages available for network formation, in turn leading to a decrease in formula viscosity, exhibited as composition thickness, while maintaining similar rheological character of the dentifrice incorporating the CMC gum system. Alternatively, in another embodiment, the use of hydroxyethyl cellulose (HEC), an uncharged water soluble cellulose derivative can remove all potential for ionic bridging and can produce dentifrice formulations of comparable thickness regardless of the presence of Zn²⁺ cations.

The present invention provides in a first aspect a dentifrice composition comprising: a. an orally acceptable vehicle; b. a zinc salt of an organic acid as a source of zinc ions, wherein the zinc salt is present in an amount of from 1 to 2.5 wt % based on the weight of the composition; c. a polysaccharide thickening agent, wherein the polysaccharide thickening agent consists of xanthan gum, and the xanthan gum is present in an amount of from 0.5 to 1.5 wt % based on the weight of the composition; d. triclosan as an antibacterial agent, and e. a methyl vinyl ether/maleic anhydride copolymer as an enhancing agent for the anti-bacterial agent.

In the invention the polysaccharide thickening agent consists of xanthan gum which is present in an amount of from 0.5 to 1.5 wt % based on the weight of the composition, preferably from 0.5 to 1 wt % of the composition.

The source of zinc ions comprises a zinc salt of an organic acid. Suitable zinc salts include zinc citrate, zinc lactate, zinc gluconate, zinc malate and zinc tartrate, typically zinc citrate. The zinc salt may be present in an amount of from 1 to 2 wt% based on the weight of the composition.

The dentifrice composition conventionally includes thickening agents that provide the dentifrice with the required rheological properties, so that the dentifrice can be stored in a dispensing container over a period of time and thereafter reliably dispensed therefrom by the user. The dentifrice must have the correct viscosity not only to be dispensed but also to exhibit an acceptable consistency within the mouth during tooth brushing. Typical thickening agents include modified celluloses, such as carboxymethyl cellulose (CMC), and other polysaccharide or gum components.

In the invention, the polysaccharide thickening agent consists of xanthan gum which is present in an amount of from 0.5 to 1.5 wt % based on the weight of the composition, preferably from 0.5 to 1 wt % of the composition. However, minor amounts of additional thickeners may be present, for example carrageenan, gum tragacanth, starch, polyvinylpyrollidione, hydroxyethypropyl cellulose, hydroxybutyl methyl cellulose, hydroxypropyl methylcellulose, hydroxyethyl cellulose, sodium carboxymethyl cellulose (sodium CMC) and colloidal silica. In one embodiment, the thickener concentration ranges of 0.1 wt. % to 5 wt. % based on the weight of the composition. In another embodiment, the thickener concentration ranges of 0.5 wt. % to 2 wt. % based on the weight of the composition.

The compositions of the present invention comprise at least one zinc ion source which is a zinc salt of an organic acid. The zinc ion source can be a soluble or a sparingly soluble compound of zinc with organic counter ions. Examples include tartrate, gluconate, citrate, malate, glycinate and oxalate salts of zinc.

Zinc ions have been found to help in the reduction of gingivitis, plaque, sensitivity, and improved breath benefits.

Zinc ions are derived from the metal ion source(s) found in the dentifrice composition in an effective amount. An effective amount is defined as from at least 1000 ppm zinc ion, preferably 2,000 ppm to 15,000 ppm. More preferably, zinc ions are present in an amount from 3,000 ppm to 13,000 ppm and even more preferably from 4,000 ppm to 10,000 ppm. This is the total amount of zinc ions that is present in the compositions for delivery to the tooth surface.

Suitable zinc ion sources are zinc citrate, zinc lactate, zinc gluconate, zinc malate and zinc tartrate.

Described herein are dentifrice compositions (outside the scope of the invention) comprising a zinc ion source(s) present in an amount of from 0.25% to 11%, by weight of the final composition. Preferably, the zinc ion sources are present in an amount of from 0.4 to 7%, more preferably from 0.45% to 5%.

A wide variety of antibacterial agents have been suggested in the art to retard plaque formation and the oral infections and dental disease associated with plaque formation. For example, halogenated hydroxydiphenyl ether compounds such as triclosan are well known to the art for their antibacterial activity and have been used in oral compositions to counter plaque formation by bacterial accumulation in the oral cavity.

Halogenated diphenyl ether antibacterial compounds that are useful for the preparation of the oral care compositions, based on considerations of antiplaque effectiveness and safety, include 2,4,4'-trichloro-2'-hydroxy-diphenyl ether (triclosan) and 2,2'-dihydroxy-5,5'-dibromo-diphenyl ether. In the invention, the antibacterial compound is 2,4,4'-trichloro-2'-hydroxydiphenyl ether ("Triclosan").

The invention also may include antibacterial compounds for oral care. Non-limiting examples of suitable antibacterial compounds include phenol and its homologs, mono and polyalkyl and aromatic halophenols, resorcinol and its derivatives and bisphenolic compounds. Such phenolic compounds are fully disclosed in U.S. Pat. No. 5,368,844. Phenolic compounds include n-hexyl resorcinol and 2,2'-methylene bis (4-chloro-6-bromophenol).

Triclosan may be present in an amount of from 0.1 to 1 wt % based on the weight of the composition, typically from 0.2 to 0.5 wt % based on the weight of the composition.

The effectiveness of the antibacterial agent is dependent upon its delivery to and uptake by teeth and soft tissue areas of the gums. The invention therefore can also contain an antibacterial agent and an adherent agent.

An antibacterial enhancing agent is also included in combination with triclosan. One particularly preferred class of antibacterial enhancing agents for triclosan includes 1:4 to 4:1 copolymers of maleic anhydride or acid with another polymerizable ethylenically unsaturated monomer. For example, one typical maleic anhydride copolymer includes a methyl vinyl ether/maleic anhydride copolymer having a molecular weight ("M.W.") ranging from 30,000 to abut 5,000,000 g/mole, or from 30,000 to 500,000 g/mole. These copolymers are commercially available, for example, under the trademark Gantrez, including Gantrez AN 139 (M.W. 500,000 g/mole), AN 119 (M.W. 250,000 g/mole); and Gantrez S-97 Pharmaceutical Grade (M.W. 700,000 g/mole), of ISP Corporation. In an aspect, the maleic anhydride copolymer typically comprises a methyl vinyl ether/maleic anhydride copolymer having a molecular weight ranging from 30,000 to abut 1,000,000 g/mole.

The invention specifically provides a dentifrice composition comprising an orally acceptable vehicle; triclosan as an antibacterial agent; a methyl vinyl ether/maleic anhydride copolymer as an enhancing agent for the antibacterial agent; a zinc salt of an organic acid as a source of zinc ions; and a polysaccharide thickening agent, the polysaccharide thickening agent consisting of xanthan gum. In an aspect, the xanthan gum is present in an amount of from 0.5 to 1.5 wt % based on the weight of the composition, the zinc salt is present in an amount of from 1 to 2.5 wt % based on the weight of the composition, and the triclosan is present in an amount of from 0.1 to 1 wt % based on the weight of the composition.

In a third aspect, there is provided a dentifrice composition as defined herein for use in a method for the treatment and prevention of bacterial plaque accumulation said method comprising: administering said dentifrice composition to the oral cavity.

The present compositions comprise essential components, as well as optional components. The essential and optional components of the compositions of the present invention are described in the following paragraphs.

In preparing the present compositions, it is desirable to add one or more aqueous carriers to the compositions. Such materials are well known in the art and are readily chosen by one skilled in the art based on the physical and aesthetic properties desired for the compositions being prepared. Aqueous carriers typically comprise from 40% to 99%, preferably from 70% to 98%, and more preferably from 90% to 95%, by weight of the dentifrice composition.

In the preparation of an oral composition in accordance with the practice of the present invention, an orally acceptable vehicle including a water-phase with humectant is present. The humectant includes one or more of glycerin, sorbitol, propylene glycol and mixtures thereof. In one embodiment, water is present in amount of at least 10 wt. % based on the weight of the composition. In another embodiment, water is present in an amount of at least 30 wt. % to 60 wt. % based on the weight of the composition. In yet another embodiment, the humectant concentration typically totals 40-60 wt. % of the oral composition.

Dentifrice compositions such as toothpastes and gels also typically contain polishing materials. In one embodiment, the polishing material includes crystalline silica, having a particle size of up to 20 microns, such as commercially available Zeodent 115, or Zeodent 165, silica gel or colloidal silica. In another embodiment, the polishing material includes compositions such as complex amorphous alkali metal aluminosilicates, hydrated alumina, sodium metaphosphate, sodium bicarbonate, calcium carbonate, calcium pyrophosphate, dicalcium phosphate and dicalcium phosphate dihydrate. In one embodiment, the polishing material is included in semi-solid or pasty dentifrice compositions, of the present invention, in an amount of 15 wt. % to 60 wt. %. In another embodiment, the composition of the present invention includes polishing material having concentrations ranging of 20 wt. % to 55 wt. % based on the weight of the composition.

The oral composition may also contain a source of fluoride ions, or fluoride-providing compound, as an anti-caries agent. In one embodiment, the fluoride ion composition is provided in an amount sufficient to supply fluoride ions ranging from 25 ppm to 5,000 ppm of the oral composition In another embodiment, the fluoride ion composition is provided in an amount sufficient to supply fluoride ions ranging from 500 to 1500 ppm of the oral composition. Representative fluoride ion providing compounds include inorganic fluoride salts, such as soluble alkali metal salts, for example, sodium fluoride, potassium fluoride, sodium fluorosilicate, ammonium flourosilicate and sodium monofluorphosphate, as well as tin fluorides, such as stannous fluoride and stannous chloride.

Any suitable flavoring or sweetening material may also be employed in the preparation of the oral compositions of the present invention. Examples of suitable flavoring constituents include flavoring oils, e.g. oil of spearmint, peppermint, wintergreen, clove, sage, eucalyptus, marjoram, cinnamon, lemon, orange, and methyl salicylate. Suitable sweetening agents include sucrose, lactose, maltose, xylitol, sodium cyclamate, aspartyl phenyl alanine methyl ester, saccharine and the like. Suitably, flavor and sweetening agents may each or together constitute 0.1 wt. % to 5 wt. % of the oral composition.

Various other materials may be incorporated in the oral preparations of this invention such as whitening agents, including urea peroxide, calcium peroxide, and hydrogen peroxide, preservatives, vitamins such as vitamin B6, B12, E and K, silicones, chlorophyll compounds and potassium salts for the treatment of dental hypersensitivity such as potassium nitrate and potassium citrate. These agents, when present, are incorporated in the compositions of the present invention in amounts which do not substantially adversely affect the properties and characteristics desired.

The dispenser for the dentifrice compositions may be a tube, pump, or any other container suitable for dispensing toothpaste.

In practicing the present invention, the user need only apply the dentifrice composition herein, to the tooth surfaces of a human or lower animal, in the areas desired, in order to obtain a desired effect, e.g., whitening, breath freshening, caries prevention, pain relief, gum health, tartar control, etc. The compositions may also be applied to other oral cavity surfaces, such as the gingival or mucosal tissues, although it is believed that the benefits are best achieved when the dentifrice compositions are applied to the teeth. The dentifrice composition may contact the tooth and/or oral cavity surface either directly, or indirectly; however, it is preferred that the dentifrice composition be directly applied. The dentifrice composition may be applied by any means, but is preferably applied with a brush or by rinsing with a dentifrice slurry.

The manufacture of the oral composition of the present invention is accomplished by any of the various standard techniques for producing such compositions. To make a dentifrice, a vehicle is prepared containing humectant, for example, one or more of glycerin, glycerol, sorbitol, and propylene glycol, thickener agents and antibacterial agent such as triclosan, and the vehicle and any surfactants are added, followed by blending in of a polishing agent, as well as fluoride salts, with the pre-mix. Finally, flavoring agent is admixed and the pH is adjusted to between 6.8 and 7.

The following examples are further illustrative of the present invention, but it is understood that the invention is not limited thereto. All amounts and proportions referred to herein and in the appended claims are by weight, unless otherwise indicated.

### EXPERIMENTAL EXAMPLES

### Example 1: Dentifrice formulations.

A number of dentifrice formulations containing zinc cations, added as zinc citrate, and triclosan, together wit h the requisite amount of the Gantrez polymer, were formulated using conventional steps and apparatus and subjected to a laboratory assessment to determine what effect the zinc cations had on the product rheology. The formulations of Samples 1 to 3 had a common amount of triclosan, 0.3 wt % based on the weight of the composition, and the thickening agent comprised xanthan gum, present in an amount of 0.8 wt % based on the weight of the composition. For the three formulations, the zinc citrate amount varied from 0.5, to 1 to 2 wt % based on the weight of the composition.

The Brookfield viscosity of the compositions was measured as follows: Product in a jar or tube is placed beneath the Brookfield Viscometer (Model RVT), which is lowered by a helipath stand at a constant rate. Measurement is allowed to proceed for 90 seconds once contact is made with the product. A recorder monitors the viscosity as a function of time. The viscosity of a dentifrice will rapidly increase (as the spindle reaches appropriate depth in the paste) and then begin to slowly increase in value. The measurement of viscosity is taken 45 seconds after this profile change is reached.

The compositions and viscosity values are shown in Table 1.

It may be seen that the Brookfield viscosity levels are quite low, below 50 Brookfield units (BFU), for each example. This is an acceptably low viscosity for a dentifrice.

Also, as the zinc level is raised there is no significant increase in viscosity. Although there is a trend towards greater viscosity, the bulk viscosity of this xanthan gum-containing formula remains acceptable even at the highest level of zinc. Also, it is known that progressive thickening is much less prevalent in xanthan formulations than in those based on cellulose thickening. Therefore these initial values for viscosity in the xanthan formula should not change appreciably over time.

### Example 2: Comparative samples 1-3

For Comparative Sample 1, as shown in Table 1 a zinc citrate/Gantrez/triclosan formula using all CMC as a gelling system has an initial viscosity greater than 100 Brookfield units. This viscosity would prevent consumers from dispensing the product and would almost certainly create problems for dispersability and mouth feel.
For Comparative Samples 2 and 3, as also shown in Table 1, a gelling system based on a ratio of CMC to Carrageenan was employed, using two different levels of zinc citrate. The viscosity was reduced below 100 Brookfield units, and so lower than for Comparative Example 1, but was still unacceptably high.

These initial values would most likely progressively thicken into an even greater viscosity problem over the course of aging. It should also be noted that in this formula base, a 2% zinc citrate formulation cannot even be considered due to the high viscosity that would result.

**Table 1**

| | Wt % zinc citrate | Wt % triclosan | Gum system | Brookfield viscosity (BFU) |
|---|---|---|---|---|
| Sample 1 | 0.5 | 0.3 | 0.8 wt % xanthan gum | 37 |
| Sample 2 | 1.0 | 0.3 | 0.8 wt % xanthan gum | 40 |
| Sample 3 | 2.0 | 0.3 | 0.8 wt % xanthan gum | 45 |
| Comparative Sample 1 | 1.0 | 0.3 | 1.1 wt % CMC | >100 |
| Comparative Sample 2 | 0.5 | 0.3 | 1.1 wt % CMC/0.5 wt % carrageenan | 66 |
| Comparative Sample 3 | 1.0 | 0.3 | 1.1 wt % CMC/0.5 wt % carrageenan | 93 |

## Claims

1. A dentifrice composition comprising:
a. an orally acceptable vehicle;
b. a zinc salt of an organic acid as a source of zinc ions, wherein the zinc salt is present in an amount of from 1 to 2.5 wt % based on the weight of the composition;
c. a polysaccharide thickening agent, wherein the polysaccharide thickening agent consists of xanthan gum, and the xanthan gum is present in an amount of from 0.5 to 1.5 wt % based on the weight of the composition;
d. triclosan as an antibacterial agent, and
e. a methyl vinyl ether/maleic anhydride copolymer as an enhancing agent for the anti-bacterial agent.

2. The composition of claim 1 wherein the source of zinc ions comprises zinc citrate.

3. The composition of claim 1 wherein triclosan is present in an amount of from 0.05 to 2 wt % based on the weight of the composition.

4. The composition of claim 3 wherein the triclosan is present in an amount of from 0.1 to 1wt % based on the weight of the composition.

5. The composition of claim 4 wherein the methyl vinyl ether/maleic anhydride copolymer has a molecular weight ranging from 30,000 to 1,000,000 g/mole.

6. A dentifrice composition according to claim 1, for use in a method for the treatment and prevention of bacterial plaque accumulation said method comprising: administering said dentifrice composition to the oral cavity.

## Patentansprüche

1. Zahnreinigungsmittelzusammensetzung, umfassend:
a. eine oral annehmbare Trägersubstanz;
b. ein Zinksalz einer organischen Säure als eine Quelle für Zinkionen, wobei das Zinksalz in einer Menge von 1 bis 2,5 Gew.-%, bezogen auf das Gewicht der Zusammensetzung, vorliegt;
c. ein Polysaccharid-Verdickungsmittel, wobei das Polysaccharid-Verdickungsmittel aus Xanthangummi besteht, und das Xanthangummi liegt in einer Menge von 0,5 bis 1,5 Gew.-%, bezogen auf das Gewicht der Zusammensetzung, vor;
d. Triclosan als ein antibakterielles Mittel, und
e. ein Methylvinylether-/Maleinsäureanhydrid-Copolymer als Verstärkungsmittel für das antibakterielle Mittel.

2. Zusammensetzung nach Anspruch 1, worin die Quelle für Zinkionen Zinkcitrat umfasst.

3. Zusammensetzung nach Anspruch 1, worin das Triclosan in einer Menge von 0,05 bis 2 Gew.-%, bezogen auf das Gewicht der Zusammensetzung, vorliegt.

4. Zusammensetzung nach Anspruch 3, worin das Triclosan in einer Menge von 0,1 bis 1 Gew.-%, bezogen auf das Gewicht der Zusammensetzung, vorliegt.

5. Zusammensetzung nach Anspruch 4, worin das Methylvinylether-/Maleinsäureanhydrid-Copolymer ein Molekulargewicht in einem Bereich von 30 000 bis 1 000 000 g/Mol hat.

6. Zahnreinigungsmittelzusammensetzung gemäß Anspruch 1 zur Verwendung in einem Verfahren zur Behandlung und Prävention von bakterieller Plaque-Akkumulation, wobei das Verfahren die Verabreichung der Zahnreinigungsmittelzusammensetzung an die Mundhöhle umfasst.

## Revendications

1. Composition de dentifrice comprenant :
a. un véhicule acceptable par voie orale ;
b. un sel de zinc d'un acide organique en tant que source d'ions zinc, dans lequel le sel de zinc est présent en une quantité de 1 à 2,5 % en poids par rapport au poids de la composition ;
c. un agent épaississant polysaccharidique, dans lequel l'agent épaississant polysaccharidique est constitué de gomme de xanthane, et la gomme de xanthane est présente en une quantité de 0,5 à 1,5 % en poids par rapport au poids de la composition ;
d. du triclosan en tant qu'agent antibactérien, et
e. un copolymère d'éther méthylvinylique/anhydride maléique en tant qu'agent d'amélioration pour l'agent antibactérien.

2. Composition selon la revendication 1, dans laquelle la source d'ions zinc comprend du citrate de zinc.

3. Composition selon la revendication 1, dans laquelle le triclosan est présent en une quantité de 0,05 à 2 % en poids par rapport au poids de la composition.

4. Composition selon la revendication 3, dans laquelle le triclosan est présent en une quantité de 0,1 à 1 % en poids par rapport au poids de la composition.

5. Composition selon la revendication 4, dans laquelle le copolymère d'éther méthylvinylique/anhydride maléique a un poids moléculaire allant de 30 000 à 1 000 000 g/mole.

6. Composition de dentifrice selon la revendication 1, pour une utilisation dans une méthode de traitement et de prévention de l'accumulation de la plaque bactérienne, ladite méthode comprenant :
l'administration de ladite composition de dentifrice à la cavité buccale.
